# EUROPÄISCHE PATENTSCHRIFT

(11) **EP 3 220 357 B1**
(45) Veröffentlichungstag und Bekanntmachung des Hinweises auf die Patenterteilung: **18.11.2026**
(21) Anmeldenummer: 16170187.5
(22) Anmeldetag: 18.05.2016
(51) Int. Cl.: G06T 15/08, G06T 17/20, G06T 7/00, G06T 19/00, A61B 6/03, A61B 6/50, G06T 7/13, G16H 50/20, A61B 6/00

(54) **ERZEUGEN UND DARSTELLEN EINER MODELLDARSTELLUNG EINES VOLUMENOBJEKTS**
GENERATION AND DISPLAY OF A MODEL REPRESENTATION OF A VOLUME OBJECT
PRODUCTION ET AFFICHAGE D'UNE REPRÉSENTATION DE MODÈLE D'UN OBJET DE VOLUME

(30) Priorität: 15.03.2016 EP 16160475
(43) Veröffentlichungstag der Anmeldung: 20.09.2017
(73) Patentinhaber: Siemens Healthineers AG, 91301 Forchheim (DE)
(72) Erfinder: Hopfgartner, Christian, 90763 Fürth (DE); Lades, Felix, 90427 Nürnberg (DE); Schwemmer, Chris, 91301 Forchheim (DE); Sühling, Michael, 91052 Erlangen (DE); Wels, Michael, 96047 Bamberg (DE)
(74) Vertreter: Siemens Healthineers Patent Attorneys

(56) Entgegenhaltungen:
- GB-A- 2 431 308
- US-A1- 2014 028 672
- VELUT J ET AL: "Assessment of qualitative and quantitative features in coronary artery MRA", IRBM, ELSEVIER, AMSTERDAM, NL, vol. 32, no. 4, 20 May 2011 (2011-05-20), pages 229 - 242, XP028389843, ISSN: 1959-0318, [retrieved on 20110607], DOI: 10.1016/J.IRBM.2011.05.002
- GUANYU YANG ET AL: "Automatic centerline extraction of coronary arteries in coronary computed tomographic angiography", THE INTERNATIONAL JOURNAL OF CARDIOVASCULAR IMAGING, 1 January 2011 (2011-01-01), XP055016051, ISSN: 1569-5794, DOI: 10.1007/s10554-011-9894-2
- ARMIN KANITSAR ET AL: "CPR", VIS 2002. IEEE VISUALIZATION 2002. PROCEEDINGS. BOSTON, MA, OCT. 27 - NOV. 1, 2002; [ANNUAL IEEE CONFERENCE ON VISUALIZATION], NEW YORK, NY : IEEE, US, 27 October 2002 (2002-10-27), pages 37 - 44, XP058097035, ISBN: 978-0-7803-7498-0
- BÜHLER K ET AL: "Geometric Methods for Vessel Visualization and Quantification - A Survey", 2003, XP002428857, Retrieved from the Internet <URL:http://www.vrvis.at/TR/2002/TR_VRVis_2002_035_Full.pdf> [retrieved on 20070410]
- KRETSCHMER JAN ET AL: "Interactive Patient-Specific Vascular Modeling with Sweep Surfaces", IEEE TRANSACTIONS ON VISUALIZATION AND COMPUTER GRAPHICS, IEEE SERVICE CENTER, LOS ALAMITOS, CA, US, vol. 19, no. 12, 1 December 2013 (2013-12-01), pages 2828 - 2837, XP011529796, ISSN: 1077-2626, [retrieved on 20131016], DOI: 10.1109/TVCG.2013.169
- WONG ET AL: "Probabilistic vessel axis tracing and its application to vessel segmentation with stream surfaces and minimum cost paths", MEDICAL IMAGE ANALYSIS, OXFORD UNIVERSITY PRESS, OXOFRD, GB, vol. 11, no. 6, 27 October 2007 (2007-10-27), pages 567 - 587, XP022318283, ISSN: 1361-8415, DOI: 10.1016/J.MEDIA.2007.05.003
- DIEPENBROCK S ET AL: "From imprecise user input to precise vessel segmentations", EG VCBM 2012 - EUROGRAPHICS WORKSHOP ON VISUAL COMPUTING FOR BIOLOGY AND MEDICINE - EG VCBM 2012 - EUROGRAPHICS WORKSHOP ON VISUAL COMPUTING FOR BIOLOGY AND MEDICINE 2012 EUROGRAPHICS ASSOCIATION DEU, 2012, pages 65 - 72, XP002776205
- JOAQUIM PEIRÃ3 ET AL: "Automatic reconstruction of a patient-specific high-order surface representation and its application to mesh generation for CFD calculations", MEDICAL & BIOLOGICAL ENGINEERING & COMPUTING, SPRINGER, BERLIN, DE, vol. 46, no. 11, 16 September 2008 (2008-09-16), pages 1069 - 1083, XP019864864, ISSN: 1741-0444, DOI: 10.1007/S11517-008-0390-3
- BRYAN S MORSE ET AL: "Interpolating implicit surfaces from scattered surface data using compactly supported radial basis functions", 20050731; 20050731 - 20050804, 31 July 2005 (2005-07-31), pages 78 - es, XP058318111, DOI: 10.1145/1198555.1198645

## Beschreibung

Die Erfindung betrifft ein Verfahren zum interaktiven Erzeugen und Verändern eines geometrischen Modells eines Volumenobjekts anhand von dreidimensionalen Bilddaten von einem Untersuchungsbereich eines Untersuchungsobjekts. Weiterhin betrifft die Erfindung eine Volumenobjekt-Modellierungseinrichtung. Schließlich betrifft die Erfindung auch eine medizinische bildgebende Einrichtung, ein Computerprogrammprodukt und ein computerlesbares Medium.

Mit Hilfe moderner bildgebender Verfahren werden häufig zwei- oder dreidimensionale Bilddaten erzeugt, die zur Visualisierung eines abgebildeten Untersuchungsobjekts und darüber hinaus auch für weitere Anwendungen genutzt werden können.

Bei der dreidimensionalen medizinischen Bildgebung müssen oft dreidimensionale Bilddaten bzw. in den Bilddaten vorhandene volumetrische geometrische Strukturen mit Hilfe eines geometrischen Modells geeignet repräsentiert werden, um Messungen vornehmen zu können oder eine Diagnose-Analyse auf einem höheren Niveau durchführen zu können. Dieser Vorgang wird im Folgenden auch als "Modellieren eines Volumenobjekts" bezeichnet. Beispiele für solche komplexeren Vorgehensweisen zu Diagnosezwecken sind das Simulieren des Blutflusses durch die Koronargefäße des Herzens durch Modellieren der Fluid-Dynamik und das Untersuchen von Ablagerungen in Fällen von Arteriosklerose. Um diese Untersuchungen vornehmen zu können, müssen die Messsignale bzw. Messdaten in eine aussagekräftige geometrische Semantik betreffend die abgebildeten Strukturen übersetzt werden. Zu diesem Zweck wurden Verfahren zur Segmentierung von medizinischen Bildaufnahmen entwickelt, mit deren Hilfe automatisiert oder interaktiv ein Bildvolumen in bekannte Objekte aufgeteilt wird. Vollautomatische Verfahren sind leider oft recht anfällig gegenüber Fehlern und daher müssen diese durch geeignete interaktive Verfahren für eine zusätzliche "Verfeinerung" der automatisiert ermittelten Segmente und damit verbundenen geometrischen Modellstrukturen ergänzt werden.

In einer beispielhaften Anwendung wird die gesamte vom Blut durchflossene Gefäßstruktur des Koronarbaums anhand von Bilddaten aus der Herz-CT-Angiographie (Cardiac Computed-Tomography Angiography = CCTA) interaktiv modelliert. Vorhandene interaktive Verfahren sind bisher nicht sehr benutzerfreundlich und sind typischerweise auf lineare planare zweidimensionale Repräsentationen der eigentlichen 3D-Daten beschränkt, wie zum Beispiel auf Darstellungen, welche durch eine multiplanare Reformatierung senkrecht zu den Mittellinien der Gefäße gewonnen wurden. Im Rahmen dieser interaktiven Modellierungen werden Oberflächenmarkierungen, wie zum Beispiel offene oder geschlossene Ansammlungen von Oberflächenpunkten, editiert bzw. markiert, um diese für eine nachfolgende Segmentierung zu nutzen. Die multiplanare Reformatierung umfasst Schnitte der dreidimensionalen Daten, in denen die Editierung vorgenommen wird. Allerdings ist der Verlauf der einzelnen Gefäße meist nicht konform zu diesen Schnitten, so dass eine Editierung anhand dieser Schnitte unvollständig ist und leicht zu falschen Ergebnissen führen kann.

In VELUT J ET AL: "Assessment of qualitative and quantitative features in coronary artery MRA", IRBM, ELSEVIER, AMSTERDAM, NL, Bd. 32, Nr. 4, 20. Mai 2011 (2011-05-20), Seiten 229-242, XP028389843, ISSN: 1959-0318, DOI: 10.1016/J.IRBM.2011.05.002) wird ein Verfahren zur Extraktion koronaler Arterienstrukturen beschrieben. Zur Darstellung der Arterie wird eine gerade ausgerichtete kurvenförmige planare Reformation (Sa-CPR) eingesetzt, wobei gewunden ausgebildete Gefäße in gestreckter Form dargestellt werden. Weiterhin erfolgt eine schichtweise Korrektur einer Mittenlinie der Arterie und anschließend eine Rekonstruktion der Gefäßwand der Arterie.

GUANYU YANG ET AL: "Automatic centerline extraction of coronary arteries in coronary computed tomographic angiography"), The INTERNATIONAL JOURNAL OF CARDIOVASCULAR IMAGING, 1. Januar 2011 (2011-01-01), XP055016051, ISSN: 1569-5794, DOI: 10.1007/s10554-011-9894-2) erfolgt in dem Abschnitt "Centerline refinement" ebenfalls eine Korrektur der "Mittenlinie", in diesem Fall mit Hilfe von MPR-Schichtbildern.

Es ist somit eine Aufgabe der vorliegenden Erfindung, ein interaktives Verfahren zur Modellierung von Volumenobjekten, insbesondere Gefäßstrukturen, anzugeben, welches eine präzisere und erleichterte Editierung von Oberflächenstrukturen für eine nachfolgende Segmentierung und modellhafte Darstellung der Volumenobjekte ermöglicht.

Diese Aufgabe wird durch ein Verfahren zum interaktiven Erzeugen eines geometrischen Modells eines Volumenobjekts gemäß Patentanspruch 1, eine Volumenobjekt-Modellierungseinrichtung gemäß Patentanspruch 9, eine medizinische bildgebende Einrichtung gemäß Patentanspruch 10, ein Computerprogramm gemäß Anspruch 11 und ein computerlesbares Medium gemäß Anspruch 12 gelöst.

Bei dem erfindungsgemäßen Verfahren zum interaktiven Erzeugen eines geometrischen Modells eines Volumenobjekts anhand von dreidimensionalen Bilddaten von einem Untersuchungsbereich eines Untersuchungsobjekts wird zunächst eine Repräsentation des Volumenobjekts auf Basis von dreidimensionalen Bilddaten ermittelt. Die Bilddaten des Volumenobjekts können beispielsweise mit einem Computertomographen oder einem Magnetresonanztomographen ermittelt worden sein, d. h. es handelt sich letztlich um Messdaten dieser Tomographiesysteme bzw. daraus rekonstruierte Bilddaten. Die Bilddaten umfassen also dreidimensionale Bilddaten bzw. einen Satz von zweidimensionalen Schichtdaten, die ein dreidimensionales Volumen abdecken.

Besonders bevorzugt handelt es sich bei den dreidimensionalen Bilddaten um medizinische Bilddaten, welche von einem Patienten gewonnen wurden.

Die Bilddaten und/oder die Repräsentation können auch aus einer Datenbank bezogen werden. Dies bedeutet, dass die Bilddaten bzw. die Repräsentation bereits vorab akquiriert bzw. ermittelt worden sind, in der Datenbank vorliegen und über eine Eingangsschnittstelle lediglich bezogen, d. h. abgerufen werden.

Unter einer Repräsentation des Volumenobjekts ist ein Datensatz zu verstehen, welcher in irgendeiner Weise die geometrische Struktur des Volumenobjekts bzw. die Position und den Verlauf des Volumenobjekts, gegebenenfalls auch nur an bestimmten ausgewählten Positionen bzw. abschnittsweise, wiedergibt. Ein Beispiel hierfür ist eine Hohlorgan-Verlaufslinie und insbesondere die später noch erläuterte Mittelliniendarstellung. Grundsätzlich kann es sich bei der Repräsentation aber auch um die gemessenen, unveränderten oder aufbereiteten (zum Beispiel gefilterten) Bilddaten selber handeln.

Weiterhin wird eine zweidimensionale Darstellung unter Anwendung einer nicht-linearen planaren Reformatierung des dreidimensionalen Objekts auf Basis der ermittelten Repräsentation erzeugt. Als nicht-linear planare Reformatierung soll eine Abbildung verstanden werden, die von einer reinen Darstellung von einzelnen Ebenen bzw. linearen Schnitten der dreidimensionalen Bilddaten, wie sie bei der multiplanaren Reformatierung (MPR) verwendet werden, abweicht. Bei der nicht-linear planaren Reformatierung werden also Bildpunkte, die nicht alle in einer Ebene liegen, zweidimensional abgebildet. Eine Art der nicht-linearen planaren Reformatierung ist die kurvenförmige bzw. gekrümmte planare Reformatierung, auch mit CPR (CPR = curved planar reformation) abgekürzt. Diese ermöglicht Darstellungen von Datensätzen, die durch einzelne geometrische Zentrallinien oder komplexe Zentralliniengraphen definiert werden. CPRs werden üblicherweise bei der Visualisierung von Gefäßen angewandt, da die erzeugten Schnitte eine sorgfältige Untersuchung der Lichtung der Gefäße erlauben und wertvollen anatomischen Kontext umfassen.

Alternativ kann auch ein in DE 10 2014 216 702.7 beschriebenes Reformatierungsverfahren angewandt werden. Dabei wird eine dreidimensionale parametrisierte Fläche festgelegt, welche konform zu einer anatomischen Struktur eines zu untersuchenden dreidimensionalen Objekts ist. Die dreidimensionale parametrisierte Fläche wird anschließend auf eine zweidimensionale parametrisierte Fläche abgebildet. Schließlich wird das zu untersuchende dreidimensionale Objekt durch Abbilden von der dreidimensionalen parametrisierten Fläche zugeordneten Bildpunkten auf die zweidimensionale parametrisierte Fläche zweidimensional dargestellt.

Bei der Ermittlung und Optimierung der zweidimensionalen parametrisierten Fläche können ARAP-Optimierungsverfahren zur Anwendung kommen. Im Gegensatz zu herkömmlichen Verfahren dient jedoch als Ausgangsfläche eine genau an die Anatomie angepasste Fläche.

Bei dieser speziellen Methode wird die Reformatierung der zu untersuchenden Bereiche durch die Anatomie von auch sehr unterschiedlichen zu untersuchenden Objekten in einem breiten Bereich bestimmt. Beispielsweise können komplexe und ausgedehnte Strukturen wie zum Beispiel Knochenskelette erfasst und so dargestellt werden, dass sie schnell untersucht werden können. Die erwähnte dreidimensionale parametrisierte Fläche kann sowohl kontinuierlich als auch diskret parametrisiert sein.

In der zweidimensionalen Darstellung erfolgt ein Editieren von Grenzflächenindikatoren, welche den Oberflächenverlauf des Volumenobjekts definieren, beispielsweise durch den Benutzer, welcher entsprechende Markierungslinien in der zweidimensionalen Darstellung einzeichnet, welche die Konturen bzw. Grenzflächen oder Grenzlinien des Volumenobjekts nachbilden. Das Einzeichnen der Grenzflächenindikatoren kann anhand von sogenannten Bildevidenzen durchgeführt werden, also Strukturen im Bild, welche zur Segmentierung genutzt werden können. Das Einzeichnen der Grenzflächenindikatoren kann aber auch, soweit die Bildevidenzen in den erfassten 3D-Bilddaten bzw. den reformatierten zweidimensionalen Bilddaten fehlen, unter Berücksichtigung des anatomischen Wissens des Benutzers vorgenommen werden. Das Editieren bzw. Einzeichnen der Markierungslinien kann auch zunächst automatisiert innerhalb des Editierprogramms auf Basis der dreidimensionalen Bilddaten und der Repräsentation erfolgen. Beispielsweise können dem Benutzer diese automatisiert ermittelten Grenzflächenindikatoren als Markierungslinien in der zweidimensionalen Darstellung angezeigt werden. Ist er mit den ermittelten Grenzflächenindikatoren einverstanden, so kann das Verfahren automatisiert fortgesetzt werden. Möchte der Benutzer jedoch an den eingezeichneten Grenzflächenindikatoren Änderungen vornehmen, so zeichnet er diese beispielsweise als Markierungslinien in der zweidimensionalen Darstellung ein. Noch einfacher lässt sich dieser Vorgang gestalten, wenn der Benutzer die automatisiert berechneten und ihm in der zweidimensionalen Darstellung angezeigten Markierungslinien der Grenzflächenindikatoren, beispielsweise auf einem Display als Bildanzeige, verschieben und in ihrer Gestalt verändern, insbesondere verzerren, strecken, stauchen, drehen usw. kann. Bei dieser teilautomatisierten Editierung wird eine Präzisierung der zunächst automatisiert bereitgestellten Grenzflächenindikatoren durch ein Eingreifen des Benutzers erzielt.

Nachfolgend wird eine dreidimensionale Darstellung der editierten Grenzflächenindikatoren durch Rücktransformieren der editierten Grenzflächenindikatoren in den dreidimensionalen Raum erzeugt. Bei der Rücktransformation wird ein Algorithmus angewandt, welcher zu dem bei dem Schritt des Erzeugens einer zweidimensionalen Bilddarstellung verwendeten Algorithmus invers ist. D. h.**,** eine Hintereinanderausführung der Reformatierung und des dazu inversen Vorgangs ergibt das Ausgangsbild. Da jedoch die Rücktransformation auf die Markierungslinien angewandt wurde, werden diese nun in den dreidimensionalen Raum übergeführt, wo sie Stützstellen für eine nachfolgende Modellierung des Volumenobjekts darstellen. Schließlich wird eine modellierte bzw. modellhafte Darstellung des Volumenobjekts auf Basis der editierten Grenzflächenindikatoren im dreidimensionalen Raum erzeugt. Beispielsweise gehen in das Ermitteln einer solchen modellhaften Darstellung Informationen bezüglich der Gestalt der Grenzflächen des darzustellenden Volumenobjekts ein. Dies kann zum Beispiel eine Information bezüglich der geometrischen Form des Querschnitts des Volumenobjekts sein. In Kombination mit den ermittelten Markierungslinien als Stützstellen kann dann die Gestalt des gesuchten Volumenobjekts rekonstruiert werden.

Ein Verfahren zur Modellierung eines Volumenobjekts auf der Basis von vorhandenen Markierungslinien ist zum Beispiel in Carr, J.C., Beatson, R.K., Cherrie, J.B., Mitchell, T.J., Fright, W.R., McCallum, B.C, and Evans, T.R.: "Reconstruction and Representation of 3D Objects with Radial Basis Functions", in: SIGGRAPH 2001: Proceedings of the 28th annual conference on computer graphics and interactive techniques, pages 67-76, 2001, beschrieben. Dabei erfolgt ein implizites Modellieren eines Volumenobjekts durch Interpolation seiner Oberfläche mittels variationaler Interpolation von radialen Basisfunktionen im dreidimensionalen Raum.

Mit dem erfindungsgemäßen Verfahren wird es Benutzern ermöglicht, Annotationen in Bilddaten, insbesondere zur Segmentierung von Volumenobjekten, interaktiv und sehr intuitiv in einer zweidimensionalen Darstellung vorzunehmen, die trotz der fehlenden dritten Dimension die Form der Volumenobjekte definierende Indikatoren vollständig umfassen. Der Benutzer kann die Markierungslinien in einer oder zwei Bilddarstellungen vollständig einzeichnen und erhält durch die reformatierte Darstellung einen Gesamtüberblick über den Verlauf dieser Markierungslinien. Anschließend ermöglicht eine automatisierte Transformation der Annotationen in den dreidimensionalen Raum eine direkte Verwendung dieser Annotationen zur Modellierung des Volumenobjekts, ohne dass der Benutzer eine Übertragung seiner Annotationen in den dreidimensionalen Raum selbst vornehmen muss.

Auf diese Weise erhält der Benutzer ein Werkzeug zum nahtlosen Editieren von Markierungslinien bzw. Grenzflächenindikatoren. Die Transformationsschritte sowie der Modellierungsschritt des Verfahrens können mit relativ geringem rechnerischen Aufwand durchgeführt werden, so dass dem Benutzer ein interaktives Verfahren mit einer fast in Echtzeit zur Verfügung stehenden Visualisierung als Rückmeldung bzw. Kontrolldarstellung bereitgestellt wird. Der Fortgang des Verfahrens ist transparent und leicht überwachbar, da Zwischenergebnisse des Verfahrens mit nur geringer Verzögerung dem Benutzer angezeigt werden können.

Die erfindungsgemäße Volumenobjekt-Modellierungseinrichtung weist eine Repräsentations-Ermittlungseinheit zum Ermitteln einer Repräsentation eines Volumenobjekts auf Basis von dreidimensionalen Bilddaten auf. Teil der erfindungsgemäßen Volumenobjekt-Modellierungseinrichtung ist zudem eine Reformatierungseinheit zum Erzeugen einer zweidimensionalen Darstellung unter Anwendung einer nicht-linear planaren Reformatierung des Volumenobjekts auf Basis der ermittelten Repräsentation. Die erfindungsgemäße Volumenobjekt-Modellierungseinrichtung umfasst außerdem eine Bildevidenz-Editierungseinheit zum Editieren von Grenzflächenindikatoren, welche den tatsächlichen Oberflächenverlauf des Volumenobjekts definieren, in der zweidimensionalen Darstellung. Überdies weist die erfindungsgemäße Volumenobjekt-Modellierungseinrichtung eine Rücktransformationseinheit auf, die dazu eingerichtet ist, eine dreidimensionale Darstellung der editierten Grenzflächenindikatoren durch Rücktransformieren der editierten Grenzflächenindikatoren in den dreidimensionalen Raum zu erzeugen. Darüber hinaus umfasst die erfindungsgemäße Volumenobjekt-Modellierungseinrichtung eine Modellierungseinheit, die dazu eingerichtet ist, das Volumenobjekt auf Basis der editierten Grenzflächenindikatoren im dreidimensionalen Raum zu modellieren bzw. eine modellhafte Darstellung des Volumenobjekts zu erzeugen.

Die erfindungsgemäße medizinische bildgebende Einrichtung, vorzugsweise ein CT-System, weist eine Scaneinheit zum Abtasten eines Untersuchungsbereichs eines zu untersuchenden Objekts, eine Steuerungseinrichtung zum Ansteuern der Scaneinheit und eine erfindungsgemäße Volumenobjekt-Modellierungseinrichtung auf.

Die Implementierung der Erfindung in ein CT-System hat die Vorteile, dass die Scan-Dauer eines CT-Systems relativ kurz ist. Sie beträgt nur wenige Sekunden im Vergleich zur Aufnahme mit MR-Systemen, welche mehrere Minuten benötigen kann. Dies ist besonders vorteilhaft bei der Untersuchung von Notfallpatienten, bei denen jede Zeitverzögerung lebensbedrohlich sein kann. Zudem sind CT-Systeme weiter verbreitet und kostengünstiger als MR-Systeme.

Die erfindungsgemäße medizinische bildgebende Einrichtung kann auch ein MR-System umfassen.

MR-Systeme dagegen haben den Vorteil, dass eine Untersuchung mit ihnen keine Röntgenstrahlenbelastung mit sich bringt und der Weichteilkontrast einer Bildaufnahme mit einem MR-System im Vergleich zu einem CT-System verbessert ist.

Die wesentlichen Komponenten der erfindungsgemäßen Volumenobjekt-Modellierungseinrichtung können zum überwiegenden Teil in Form von Softwarekomponenten ausgebildet sein. Dies betrifft insbesondere die Repräsentations-Ermittlungseinheit, die Reformatierungseinheit, die Bildevidenz-Editierungseinheit, die Rücktransformationseinheit und die Modellierungseinheit. Grundsätzlich können diese Komponenten aber auch zum Teil, insbesondere wenn es um besonders schnelle Berechnungen geht, in Form von softwareunterstützter Hardware, beispielsweise FPGAs oder dergleichen, realisiert sein. Ebenso können die benötigten Schnittstellen, beispielsweise wenn es nur um eine Übernahme von Daten aus anderen Softwarekomponenten geht, als Softwareschnittstellen ausgebildet sein. Sie können aber auch als hardwaremäßig aufgebaute Schnittstellen ausgebildet sein, die durch geeignete Software angesteuert werden.

Eine weitgehend softwaremäßige Realisierung hat den Vorteil, dass auch schon bisher verwendete Steuereinrichtungen von medizinischen bildgebenden Einrichtungen auf einfache Weise durch ein Software-Update nachgerüstet werden können, um auf die erfindungsgemäße Weise zu arbeiten. Insofern wird die Aufgabe auch durch ein entsprechendes Computerprogrammprodukt mit einem Computerprogramm gelöst, welches direkt in eine Speichereinrichtung einer Steuereinrichtung einer medizinischen bildgebenden Einrichtung ladbar ist, mit Programmabschnitten, um alle Schritte des erfindungsgemäßen Verfahrens auszuführen, wenn das Programm in der Steuereinrichtung ausgeführt wird. Ein solches Computerprogrammprodukt kann neben dem Computerprogramm gegebenenfalls zusätzliche Bestandteile wie z. B. eine Dokumentation und/oder zusätzliche Komponenten, auch Hardware-Komponenten, wie z. B. Hardware-Schlüssel (Dongles etc.) zur Nutzung der Software, umfassen.

Zum Transport zur Steuereinrichtung und/oder zur Speicherung an oder in der Steuereinrichtung kann ein computerlesbares Medium, beispielsweise ein Memorystick, eine Festplatte oder ein sonstiger transportabler oder fest eingebauter Datenträger dienen, auf welchem die von einer Rechnereinheit der Steuereinrichtung einlesbaren und ausführbaren Programmabschnitte des Computerprogramms gespeichert sind. Die Rechnereinheit kann hierzu z. B**.** einen oder mehrere zusammenarbeitende Mikroprozessoren oder dergleichen aufweisen.

Die abhängigen Ansprüche sowie die nachfolgende Beschreibung enthalten jeweils besonders vorteilhafte Ausgestaltungen und Weiterbildungen der Erfindung. Dabei können insbesondere die Ansprüche einer Anspruchskategorie auch analog zu den abhängigen Ansprüchen einer anderen Anspruchskategorie weitergebildet sein. Zudem können im Rahmen der Erfindung auch die verschiedenen Merkmale unterschiedlicher Ausführungsbeispiele und Ansprüche auch zu neuen Ausführungsbeispielen kombiniert werden.

In dem erfindungsgemäßen Verfahren zum interaktiven Erzeugen eines geometrischen Modells eines Volumenobjekts anhand von dreidimensionalen Bilddaten von einem Untersuchungsbereich eines Untersuchungsobjekts weist das Volumenobjekt eine röhrenartige Struktur auf. Eine solche röhrenartige Struktur kann zum Beispiel eine dreidimensionale Gefäßstruktur, vorzugsweise eine Koronargefäßstruktur, umfassen. Derartige weitverzweigte feine Gefäßstrukturen sind schwer allein automatisiert anhand von dreidimensionalen Bilddaten zu modellieren. Insbesondere, wenn Blutflussberechnungen vorgenommen werden sollen, ist es jedoch sehr wichtig, dass die modellhaft repräsentierten Gefäße fluiddicht sind und einigermaßen exakt rekonstruiert werden. Mit dem erfindungsgemäßen Verfahren werden die Vorteile einer automatisierten Modellierung solcher Gefäßstrukturen mit den Möglichkeiten eines korrigierenden Eingreifens des Benutzers kombiniert, so dass ein präziseres Ergebnis als bei einem Vorgehen nur von Hand oder nur automatisiert erreicht wird.

In dem erfindungsgemäßen Verfahren umfasst die Repräsentation eine Mittellinie, auch Centerline genannt, längs des Verlaufs der röhrenartigen Struktur. Sie repräsentiert den Verlauf des betreffenden Hohlorgans zumindest grob. Die Mittellinie kann beispielsweise manuell durch einen Benutzer und/oder automatisch durch einen Ermittlungsalgorithmus ermittelt werden. Auch eine halbautomatische, d. h. automatische - jedoch durch einen Benutzer assistierte - Ermittlung ist möglich. Eine Methode zur Ermittlung einer solchen Mittellinie in einer Gefäßstruktur ist in Zheng, Y., Shen, J., Tek, H., Funka-Lea, G.: "Model-Driven Centerline Extraction for Severely Occluded Major Coronary Arteries", in: Machine Learning in Medical Imaging, LNCS 7588, pages 10-18, 2012, beschrieben. Da die Mittellinie den Verlauf der röhrenartigen Strukturen im dreidimensionalen Raum wiedergibt, wird diese dazu genutzt, eine Fläche im dreidimensionalen Raum für die spätere Reformatierung festzulegen, welche die Röhrenwände in ihrem Verlauf längs der Mittellinie ebenfalls umfasst. Auf diese Weise wird eine nahtlose Nachverfolgung der Röhrenwände in der reformatierten zweidimensionalen Darstellung ermöglicht, welche das Auffinden und Annotieren dieser Röhrenwände erleichtert.

In einer alternativen Variante des erfindungsgemäßen Verfahrens markieren die editierten Grenzflächenindikatoren den Verlauf der Röhrenwände der röhrenartigen Struktur. D. h., die Markierungslinien markieren den Verlauf der Röhrenwände und definieren so die Grenzen eines bei einer späteren Untersuchung zu ermittelnden Hohlvolumens, allerdings zunächst noch im zweidimensionalen Raum.

In einer besonders effektiven Ausgestaltung des erfindungsgemäßen Verfahrens basiert die Rücktransformation der markierten zweidimensionalen Darstellung in den dreidimensionalen Raum auf einer Invertierung der nicht-linear planaren Reformatierung. Da die Modellierung des Volumenobjekts im dreidimensionalen Raum erfolgen muss, werden die editierten Annotationen durch Invertierung der vorhergehenden Reformatierung wieder in den dreidimensionalen Raum zurücktransformiert. Da die Rücktransformation einer Invertierung der vorhergehenden Reformatierung entspricht, ist gewährleistet, dass die Markierungslinien bzw. editierten Grenzflächenindikatoren von der Form her konform mit dem tatsächlichen strukturellen Aufbau des Volumenobjekts sind. Mithin wird eine präzise und formtreue Modellierung des zu untersuchenden Volumenobjekts erreicht.

In einer Variante des Verfahrens werden in der dreidimensionalen Darstellung der editierten Grenzflächenindikatoren früher eingezeichnete widersprüchliche Grenzflächenindikatoren, welche durch einen vorbestimmten Volumenbereich um eine aktuell eingezeichnete Markierungslinie herum verlaufen, verworfen. Beispielsweise kann diese Vorgehensweise im Fall der Untersuchung eines Hohlorgans oder röhrenförmigen Objekts dahingehend implementiert sein, dass ältere Markierungslinien bzw. Grenzflächenindikatoren, die in einem Volumenbereich um eine neue Markierungslinie liegen, der durch einen Stapel aus Kreisringsegmenten um die Mittellinie des Hohlorgans mit einem vorbestimmten Winkel und in einem vorbestimmten Abstand zu der Mittellinie angeordnet ist, verworfen werden. Auf diese Weise werden falsch oder ungenau annotierte Markierungslinien durch präziser eingezeichnete Markierungslinien ersetzt, wobei in Folge der Iteration eine allmähliche Konvergenz zu einem Optimum, was die Präzision der Markierungslinien angeht, erreicht wird.

Konkret bedeutet das, dass die Schritte des Editierens der Grenzflächenindikatoren und des Verwerfens von widersprüchlichen Grenzflächenindikatoren bzw. Markierungslinien in einem iterativen Vorgang abwechselnd durchgeführt werden, bis alle Grenzflächenindikatoren editiert sind und alle widersprüchlichen Grenzflächenindikatoren eliminiert sind. Bei dieser Variante erfolgt schrittweise eine Verfeinerung der eingezeichneten Annotationen bzw. Markierungslinien, wobei ältere Markierungslinien, die aktuell eingezeichneten Markierungslinien "im Weg stehen" bzw. diesen zu nahe kommen, verworfen werden.

Bevorzugt umfasst das Modellieren des Volumenobjekts auf Basis der editierten Grenzflächenindikatoren ein implizites Modellieren durch Interpolieren der Oberflächen des Volumenobjekts mit radialen Basisfunktionen, wobei sogenannte implizite Flächen definiert werden. Implizite Flächen erlauben ein sehr flexibles, "modellfreies" Modellieren von Grenzflächen ohne Einschränkung auf bestimmte Grundformen der Grenzflächen. Mithin wird eine erhöhte Flexibilität und Genauigkeit bei der Ermittlung der Grenzflächen eines Volumenobjekts erzielt.

Besonders bevorzugt wird das modellierte Volumenobjekt durch ein Flächennetz im dreidimensionalen Raum und/oder durch in der reformatierten Fläche der zweidimensionalen Darstellung liegende Schnittlinien bildlich dargestellt. Bei letzterer Darstellungsweise betrachtet der Benutzer eine dem Verlauf des Volumenobjekts folgende reformatierte Fläche und eine Liniendarstellung bzw. Schnittliniendarstellung der diese reformatierte Fläche schneidenden modellierten Grenzflächen des Volumenobjekts. Die Schnittliniendarstellung ergibt sich daraus, dass die dem Verlauf des Volumenobjekts folgende gekrümmte Fläche implizite Oberflächen des modellhaft erzeugten Volumenobjekts schneidet, die bei dieser Variante in einer zweidimensionalen Darstellung als Schnittlinien abgebildet werden.

Für den Fall einer Darstellung mit Schnittliniendarstellung können diese Schnittlinien für eine Verfeinerung des Modells als Kandidaten-Grenzflächenindikatoren bei einem im Rahmen einer Iteration erneut ausgeführten Schritt des Editierens von Grenzflächenindikatoren verwendet werden. D. h., das ermittelte Volumenobjekt kann in seiner Projektionsdarstellung selbst wieder als Markierungslinien in der zweidimensionalen Darstellung genutzt werden, die dann vom Benutzer in der zweidimensionalen Darstellung noch nachkorrigiert werden können, um so eine Verbesserung der Genauigkeit der Ermittlung der Grenzflächen des Volumenobjekts zu erreichen.

Die Erfindung wird im Folgenden unter Hinweis auf die beigefügten Figuren anhand von Ausführungsbeispielen noch einmal näher erläutert. Es zeigen:
- FIG 1: ein Flussdiagramm, welches ein Verfahren zum interaktiven geometrischen Modellieren eines Volumenobjekts anhand von dreidimensionalen Bilddaten von einem Untersuchungsbereich eines Untersuchungsobjekts veranschaulicht,
- FIG 2: Abbildungen eines Herzens, welche eine Festlegung von Zentrallinien der Herzkranzgefäße veranschaulichen,
- FIG 3: eine schematische Darstellung eines Schritts einer Editierung des Verlaufs von Gefäßoberflächen in einer reformatierten zweidimensionalen Darstellung von Herzkranzgefäßen,
- FIG 4: eine dreidimensionale Darstellung des Verlaufs von editierten Verlaufslinien der Gefäßoberflächen,
- FIG 5: eine zweidimensionale Darstellung einer modellierten Gefäßstruktur eines Herzkranzgefäßes,
- FIG 6: eine dreidimensionale Darstellung eines modellierten Herzkranzgefäßes in einem Koronargefäßbaum,
- FIG 7: eine dreidimensionale Darstellung einer Mehrzahl von miteinander verbundenen modellierten Herzkranzgefäßen in einem Koronargefäßbaum,
- FIG 8: ein Blockdiagramm, mit dem eine Volumenobjekt-Modellierungseinrichtung gemäß einem Ausführungsbeispiel der Erfindung veranschaulicht wird.

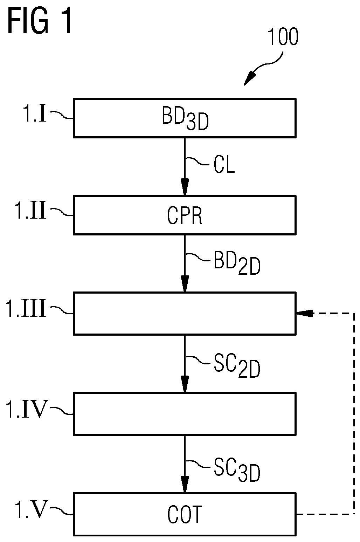

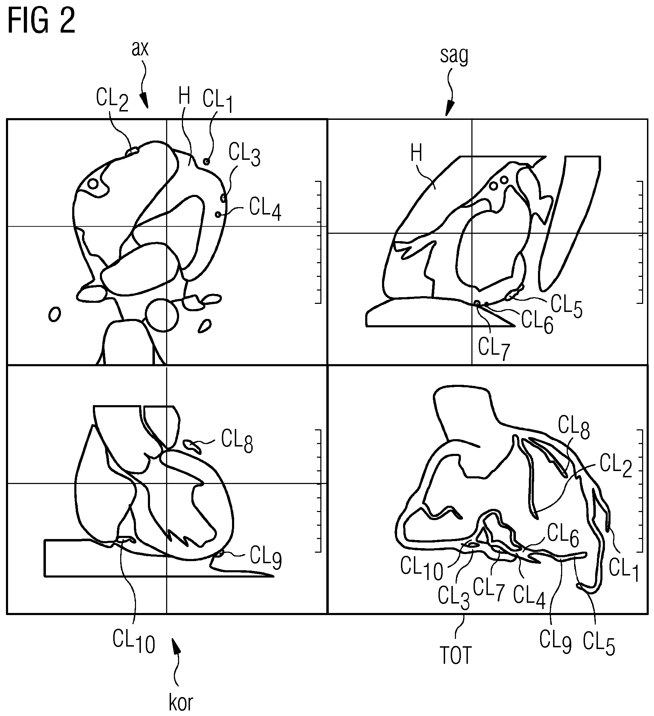

In FIG 1 ist ein Flussdiagramm 100 gezeigt, welches ein Verfahren zum interaktiven geometrischen Modellieren eines Volumenobjekts anhand von dreidimensionalen Bilddaten von einem Untersuchungsbereich eines Untersuchungsobjekts gemäß einem Ausführungsbeispiel der Erfindung veranschaulicht. Bei dem Verfahren werden Gefäßstrukturen eines Koronarbaums modelliert, welche nachfolgend für verschiedene Arten von Berechnungen für die Diagnose, beispielsweise betreffend den Blutfluss durch die Gefäße, verwendet werden können. Das mit dem Flussdiagramm in FIG 1 beschriebene Verfahren wird zusätzlich mit Hilfe von FIG 2 bis FIG 7 erläutert, in denen zu den einzelnen Verfahrensschritten Illustrationen gezeigt sind, die die einzelnen Schritte im Detail veranschaulichen. Bei dem Schritt 1.I werden zunächst dreidimensionale Daten BD_{3D} empfangen, die zum Beispiel von einer kardialen CT-Angiographie (CTA) stammen. Um auf Basis dieser Daten BD_{3D} quantitative Untersuchungen bezüglich der Funktionsfähigkeit des Koronarsystems vornehmen zu können, müssen die Bilddaten segmentiert werden. Beispielsweise kann damit eine Abgrenzung der Gefäße gegen die Umgebung vorgenommen werden und so zum Beispiel ein Volumen und Querschnitte des Gefäßsystems und daraus ein maximaler Durchfluss durch das Gefäßsystem ermittelt werden. Zunächst wird bei dem Schritt 1.I eine Zentrallinie CL der Gefäße in den Bilddaten BD_{3D} festgelegt. Diese Zentrallinie oder Mittellinie CL kann als Repräsentation der Gefäßstrukturen verstanden werden. Sie bestimmt den Verlaufsweg der in den Bilddaten BD_{3D} erfassten Gefäßstrukturen.

In FIG 2 sind drei verschiedene zweidimensionale Schnittdarstellungen ax, sag, kor des Herzens H sowie zusätzlich in einer Teilzeichnung unten rechts eine Gesamtdarstellung TOT des Koronarbaums gezeigt. In einer Teilzeichnung ax oben links ist eine axiale Darstellung des Herzens H gezeigt. In der axialen Darstellung ax werden Positionen CL₁, CL₂, CL₃, CL₄ ermittelt, welche als Zentralpunkte von Koronargefäßen anzusehen sind. Ebenso werden in einer Teilzeichnung sag oben rechts, welche eine sagittale Ansicht des Herzens H zeigt, Positionen CL₅, CL₆, CL₇ identifiziert, welche Zentralpunkte der Koronargefäße des Herzens darstellen. Als Zentralpunkte sind in diesem Zusammenhang Punkte auf einer Zentrallinie CL eines Gefäßes zu verstehen. In einer Teilzeichnung kor unten links ist in einer koronalen Richtung eine Darstellung des Herzens H gezeigt, in der wiederum Zentralpunkte CL₈, CL₉, CL₁₀ markiert sind, welche ebenfalls zu Blutgefäßen des Koronarbaums des Herzens gehören. In einer Teilzeichnung TOT unten rechts ist eine perspektivische Darstellung des gesamten Koronarbaums dargestellt. In dem Koronarbaum sind auch die Zentralpunkte CL₁, ..., CL₁₀ aus den drei Schnittdarstellungen ax, sag, kor eingezeichnet. Bei einer entsprechend großen Anzahl von untersuchten Schnittdarstellungen können mithin durch Verbindung der Zentralpunkte Mittellinien CL der Koronargefäße ermittelt werden. In der Teilzeichnung TOT unten rechts wird tatsächlich nur "dargestellt" (durch geeignete Fensterung und Maskierung) und eben nicht wie gemäß der Erfindung mit kontinuierlichen Grenzflächen geometrisch modelliert.

Bei einem Schritt 1.II (siehe FIG 1) wird nach der Ermittlung der Mittellinien CL der Blutgefäße des Herzens eine zweidimensionale Darstellung BD_{2D} auf Basis der dreidimensionalen Bilddaten BD_{3D} erzeugt. Hierfür wird eine sogenannte nicht-linear planare Reformatierung bzw. in diesem speziellen Fall eine kurvenförmige bzw. gekrümmte Reformatierung CPR der dreidimensionalen Bilddaten BD_{3D} vorgenommen. Anschaulich gesprochen, erfolgt eine Art Abbildung eines Teils der dreidimensionalen Bilddaten BD_{3D}, nämlich einer nicht-planaren Fläche, welche die Mittellinien CL der Koronargefäße umfasst, in den zweidimensionalen Raum. Auf diese Weise wird garantiert, dass im Gegensatz zu einer multiplanaren Reformatierung auch in der zweidimensionalen Darstellung BD_{2D} die Gefäßstrukturen um die Mittellinien herum nahtlos abgebildet werden.

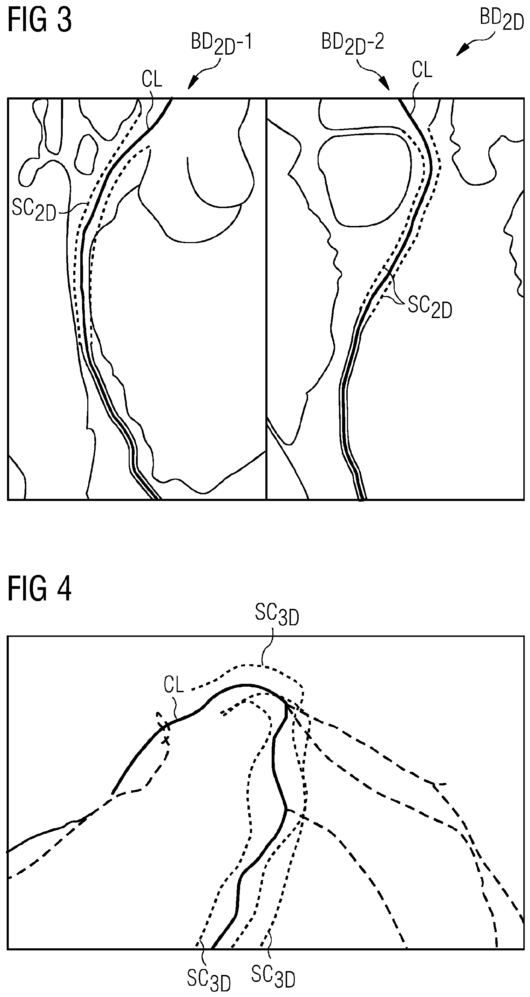

Zwei solche durch Reformatierung gewonnene Darstellungen BD_{2D}-1, BD_{2D}-2 von Gefäßstrukturabschnitten des Herzens sind in FIG 3 gezeigt. Dabei sind die beiden Darstellungen Ansichten, welche zueinander um 90° gedreht sind. In FIG 3 ist in der ersten Darstellung BD_{2D}-1 eine Mittellinie CL eingezeichnet, welche den Längsverlauf eines Koronargefäßes markiert. In einer um 90° gedrehten Ansicht BD_{2D}-2 ist ebenfalls eine Mittellinie CL desselben Koronargefäßes eingezeichnet, nun aus einer um 90° gedrehten Perspektive betrachtet.

Bei einem Schritt 1.III (siehe FIG 1) erfolgt nun ein Editieren von Grenzflächenindikatoren SC_{2D}, in diesem Fall von Berandungslinien, welche den tatsächlichen Oberflächenverlauf der Koronargefäße markieren, in der zweidimensionalen Darstellung BD_{2D}. Diese Markierungslinien SC_{2D} sind ebenfalls in FIG 3 in den beiden Teilzeichnungen BD_{2D}-1, BD_{2D}-2 eingezeichnet. Wie in FIG 3 zu erkennen ist, kann eine "zweidimensionalen Darstellung" BD_{2D} auch mehrere solche Darstellungen BD_{2D}-1, BD_{2D}-2 umfassen, die eine Koronarstruktur aus verschiedenen Richtungen betrachtet veranschaulichen. Der Verlauf der Markierungslinien SC_{2D} kann zum Beispiel vom Benutzer anhand von Kontrastunterschieden in der zweidimensionalen Darstellung BD_{2D} und auch unter Nutzung seines anatomischen Wissens und seiner ärztlichen Praxis festgelegt werden.

Nachfolgend werden bei einem Schritt 1.IV die festgelegten Mittellinien CL und die Markierungslinien SC_{2D} in den dreidimensionalen Raum zurücktransformiert, wobei nun eine im Vergleich zu der bei dem Schritt 1.II angewandten Transformation inverse Transformation angewandt wird. Die Reformatierung bei dem Schritt 1.II wird quasi rückgängig gemacht, wobei allerdings bei dem Schritt 1.IV nur die Mittellinien CL und die Markierungslinien SC_{2D} invers in den dreidimensionalen Raum transformiert werden. Bei dem Schritt 1.IV werden also dreidimensionale Markierungslinien SC_{3D} auf Basis der zweidimensionalen Markierungslinien SC_{2D} erzeugt. Sind wie in FIG 3 veranschaulicht, zweidimensionale Ansichten aus zwei verschiedenen Richtungen vorhanden, so sind die dreidimensionalen Markierungslinien SC_{3D} durch die in den zweidimensionalen Ansichten eingezeichneten zweidimensionalen Markierungslinien SC_{3D} eindeutig definiert. Diese Markierungslinien SC_{3D} sind gemeinsam mit Mittellinien CL in FIG 4 in einer dreidimensionalen Darstellung veranschaulicht. Diese Markierungslinien SC_{3D}, CL können nachfolgend als Stützstellen für eine Modellierung von Gefäßstrukturen COT genutzt werden.

Bei dem Schritt 1.V erfolgt eine solche Modellierung eines Koronargefäßabschnitts COT auf Basis der bei dem Schritt 1.IV gewonnenen dreidimensionalen Markierungslinien SC_{3D} sowie der bereits bei dem Schritt 1.I erhaltenen Mittellinien CL. Die Modellierung kann zum Beispiel als implizite Modellierung mit Hilfe radialer Basisfunktionen erfolgen, wobei die Oberfläche der Gefäße unter Anwendung eines Interpolationsverfahrens im dreidimensionalen Raum erzeugt wird. Die dabei gewonnenen impliziten Flächen können wiederum als explizite Flächen zum Beispiel durch dreidimensionale Flächennetze in einer dreidimensionalen Darstellung oder als sich überschneidende zweidimensionale Linien in reformatierter Form in einer zweidimensionalen Darstellung veranschaulicht werden.

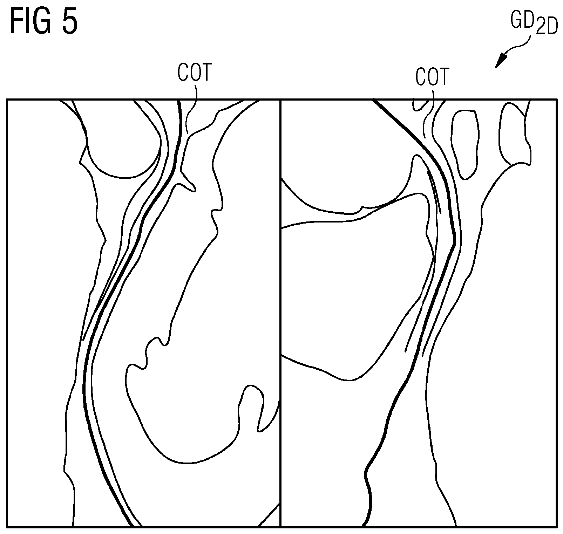

Eine solche zweidimensionale Darstellung GD_{2D} ist in FIG 5 gezeigt, wobei analog zu FIG 3 zwei um 90° zueinander gedrehte Ansichten der Gefäßstrukturen COT veranschaulicht sind. Die die Gefäßstrukturen repräsentierenden Linien COT können auch in einem Korrekturschritt von einem Benutzer geändert werden und anschließend per inverser Reformatierung wieder in den dreidimensionalen Raum zurücktransformiert werden, um eine verbesserte Modelldarstellung der Gefäßstrukturen COT zu erhalten. Diese optionale Vorgehensweise ist in FIG 1 durch einen gestrichelten Pfeil zwischen dem Schritt 1.V und dem Schritt 1.III eingezeichnet. Nachfolgend können bei dem Schritt 1.III neue Markierungslinien SC_{2D} eingezeichnet werden, die ältere Markierungslinien ersetzen. Die "ungenauen" älteren Markierungslinien brauchen dabei nicht unbedingt vom Benutzer von Hand entfernt zu werden. Es können zum Beispiel die älteren Markierungslinien auch zunächst bei dem Schritt 1.IV in den dreidimensionalen Raum mittransformiert werden und anschließend ein automatisierter Aussortiervorgang erfolgen, bei dem ältere Markierungslinien, die in einem Volumenbereich um eine neue Markierungslinie liegen, der durch einen Stapel aus Kreisringsegmenten um die Mittellinie mit einem vorbestimmten Winkel und in einem vorbestimmten Abstand zu der Mittellinie angeordnet ist, verworfen werden. Schließlich erfolgt bei dem Schritt 1.V wieder eine Modellierung der Gefäßstrukturen COT, nun auf Basis der korrigierten Markierungslinien.

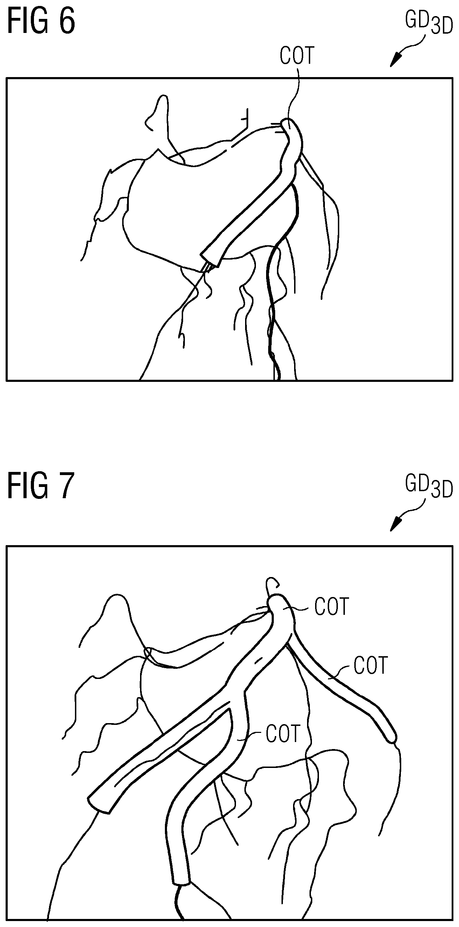

In FIG 6 ist eine dreidimensionale Darstellung GD_{3D} einer modellierten Gefäßstruktur COT gezeigt, welche durch ein Gitternetz repräsentiert wird.

Das im Zusammenhang mit FIG 1 bis FIG 6 beschriebene Verfahren kann mit einer Modelldarstellung von weiteren Gefäßabschnitten des in den Angiographiedaten BD_{3D} abgebildeten Koronarbaums fortgesetzt werden. Eine solche Fortsetzung des Verfahrens ist in FIG 7 veranschaulicht, wobei weitere Zweige COT des Koronarbaums durch Gitternetze modelliert sind. Zusätzlich kann auch an den Übergängen zwischen den einzelnen Gefäßzweigen, auch als Bifurkation bezeichnet, eine Vereinigung von impliziten Flächen, welche unterschiedlichen Mittellinien zugeordnet sind, erfolgen. Das Ergebnis einer solchen allgemeinen Modellierung eines größeren Abschnitts des Koronarbaums ist in FIG 7 zu erkennen.

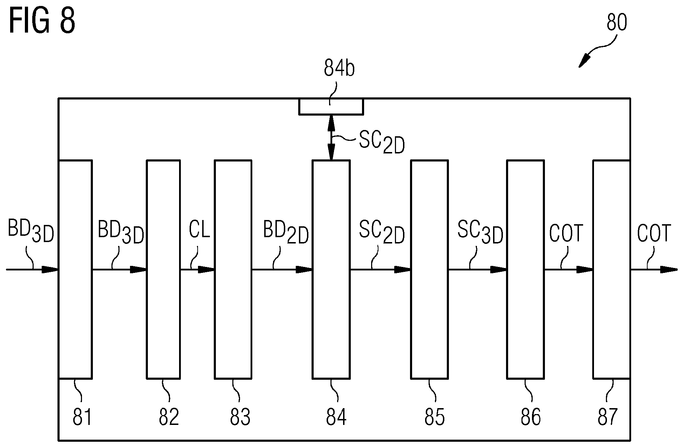

In FIG 8 ist ein Blockdiagramm gezeigt, welches eine Volumenobjekt-Modellierungseinrichtung 80 gemäß einem Ausführungsbeispiel der Erfindung veranschaulicht. Die Volumenobjekt-Modellierungseinrichtung 80 umfasst eine Eingangsschnittstelle 81, welche dreidimensionale Bilddaten BD_{3D} umfasst, die eine Abbildung des Herzens darstellen. Die erfassten dreidimensionalen Bilddaten BD_{3D} werden an eine Repräsentations-Ermittlungseinheit 82 übermittelt, welche auf Basis der dreidimensionalen Bilddaten BD_{3D} Mittellinien CL der Herzkranzgefäße ermittelt und in den dreidimensionalen Bilddaten BD_{3D} markiert. Die so markierten dreidimensionalen Bilddaten BD_{3D} werden an eine Reformatierungseinheit 83 übermittelt, die daraus eine zweidimensionale Darstellung BD_{2D} unter Anwendung einer nicht-linear planaren Reformatierung CPR auf Basis der ermittelten Mittellinien CL erzeugt. Die auf diese Weise erzeugten Bilddaten BD_{2D} einer zweidimensionalen Darstellung werden an eine Bildevidenz-Editierungseinheit 84 weitergesandt. Die Bildevidenz-Editierungseinheit 84 dient einer Editierung von Grenzflächenindikatoren SC_{2D}, welche den tatsächlichen Oberflächenverlauf der Gefäßstrukturen COT definieren, in der zweidimensionalen Darstellung BD_{2D}. Hierzu ist die Bildevidenz-Editierungseinheit 84 an eine Kommunikationsschnittstelle 84b angeschlossen, über die ein Benutzer Markierungslinien als Grenzflächenindikatoren SC_{2D} in der zweidimensionalen Darstellung einzeichnen kann, welche dann von der Bildevidenz-Editierungseinheit 84 übernommen werden. Anschließend werden die interaktiv durch den Benutzer erzeugten Markierungslinien SC_{2D} bzw. diesen entsprechende Bilddaten an eine Rücktransformationseinheit 85 übermittelt, die eine dreidimensionale Darstellung SC_{3D} der editierten Markierungslinien SC_{2D} durch Rücktransformieren der editierten Markierungslinien SC_{2D} in den dreidimensionalen Raum erzeugt. Die erzeugten Daten SC_{3D}, umfassend dreidimensionale Markierungslinien, werden schließlich an eine Modellierungseinheit 86 übermittelt, welche auf Basis der editierten Markierungslinien SC_{3D} im dreidimensionalen Raum Gefäßstrukturen COT modelliert. Die Bilddaten mit den modellierten Gefäßstrukturen COT werden dann über eine Ausgangsschnittstelle 87 an andere Einheiten - wie zum Beispiel eine Bilddatenspeichereinheit - ausgegeben, um zum Beispiel auf einem Bildschirm dem Benutzer angezeigt zu werden oder für eine weiterführende Analyse für eine Befundung als Datenbasis zu dienen.

Es wird abschließend noch einmal darauf hingewiesen, dass es sich bei den vorbeschriebenen Verfahren und Vorrichtungen lediglich um bevorzugte Ausführungsbeispiele der Erfindung handelt und dass die Erfindung vom Fachmann variiert werden kann, ohne den Bereich der Erfindung zu verlassen, soweit er durch die Ansprüche vorgegeben ist. Die Erfindung ist nicht auf eine Anwendung im medizinischen Bereich beschränkt, sondern die Erfindung kann auch grundsätzlich auf die Ermittlung von Volumenobjekten anhand von Bilddaten angewandt werden. Es wird der Vollständigkeit halber auch darauf hingewiesen, dass die Verwendung der unbestimmten Artikel "ein" bzw. "eine" nicht ausschließt, dass die betreffenden Merkmale auch mehrfach vorhanden sein können. Ebenso schließt der Begriff "Einheit" nicht aus, dass diese aus mehreren Komponenten besteht, die gegebenenfalls auch räumlich verteilt sein können.

## Patentansprüche

1. Verfahren zum interaktiven Erzeugen eines geometrischen Modells eines Volumenobjekts (COT) anhand von dreidimensionalen Bilddaten (BD_{3D}) von einem Untersuchungsbereich eines Untersuchungsobjekts, wobei das Volumenobjekt (COT) eine röhrenartige Struktur aufweist, aufweisend die Schritte:
- Ermitteln einer Repräsentation (CL) des Volumenobjekts (COT) auf Basis von dreidimensionalen Bilddaten (BD_{3D}) des Volumenobjekts (COT), wobei die Repräsentation (CL) des Volumenobjekts (COT) eine Mittellinie längs des Verlaufs der röhrenartigen Struktur umfasst,
- Erzeugen einer zweidimensionalen Darstellung (BD_{2D}) unter Anwendung einer nicht-linear planaren Reformatierung (CPR) des Volumenobjekts (COT) auf Basis der ermittelten Repräsentation (CL), wobei die Mittellinie des Volumenobjekts dazu genutzt wird, eine Fläche im dreidimensionalen Raum für die Reformatierung festzulegen, welche die Röhrenwände der röhrenartigen Struktur in ihrem Verlauf längs der Mittellinie ebenfalls umfasst,
- Editieren von Grenzflächenindikatoren (SC_{2D}), welche den Oberflächenverlauf des Volumenobjekts (COT) definieren, in der zweidimensionalen Darstellung (BD_{2D}), wobei Grenzflächenindikatoren einem Benutzer als Markierungslinien in der zweidimensionalen Darstellung angezeigt werden, wobei ein Benutzer zum Editieren von Grenzflächenindikatoren (SC_{2D}) Änderungen als Markierungslinien in der zweidimensionalen Darstellung einzeichnet oder in der zweidimensionalen Darstellung dargestellte Markierungslinien verschiebt und in ihrer Gestalt verändert,
- Erzeugen einer dreidimensionalen Darstellung (SC_{3D}) der editierten Grenzflächenindikatoren (SC_{2D}) durch Rücktransformieren der in der zweidimensionalen Darstellung editierten Grenzflächenindikatoren (SC_{2D}) in den dreidimensionalen Raum,
- Ermitteln einer geometrischen Modelldarstellung des Volumenobjekts (COT) auf Basis der durch Rücktransformieren erzeugten dreidimensionalen Darstellung der editierten Grenzflächenindikatoren (SC_{2D}) im dreidimensionalen Raum.

2. Verfahren nach Anspruch 1, wobei das Volumenobjekt (COT) ein Hohlorgan aufweist.

3. Verfahren nach Anspruch 2, wobei die röhrenartige Struktur eine dreidimensionale Gefäßstruktur, vorzugsweise eine Koronargefäßstruktur, umfasst.

4. Verfahren nach Anspruch 2 oder 3, wobei die Mittellinie (CL) den Verlauf der röhrenartigen Struktur definiert.

5. Verfahren nach einem der Ansprüche 2 bis 4, wobei die editierten Grenzflächenindikatoren (SC_{2D}, SC_{3D}) den Verlauf von Röhrenwänden der röhrenartigen Struktur markieren.

6. Verfahren nach einem der Ansprüche 1 bis 5, wobei die Rücktransformation der editierten Grenzflächenindikatoren (SC_{2D}) aus der zweidimensionalen Darstellung (BD_{2D}) in den dreidimensionalen Raum auf einer Invertierung der nicht-linear planaren Reformatierung (CPR) basiert.

7. Verfahren nach einem der Ansprüche 1 bis 6, wobei das modellierte Volumenobjekt (COT) durch ein Flächennetz im dreidimensionalen Raum und/oder durch in der reformatierten Fläche der zweidimensionalen Darstellung (BD_{2D}) liegende Schnittlinien bildlich dargestellt wird.

8. Verfahren nach Anspruch 7, wobei für den Fall einer Modelldarstellung des Volumenobjekts (COT) mit zweidimensionalen, in der reformatierten Fläche liegenden Schnittlinien diese Schnittlinien für eine Verfeinerung des Modells als Kandidaten-Grenzflächenindikatoren bei einem im Rahmen einer Iteration erneut ausgeführten Schritt des Editierens von Grenzflächenindikatoren (SC_{2D}) verwendet werden.

9. Volumenobjekt-Modellierungseinrichtung (80), aufweisend:
- eine Repräsentations-Ermittlungseinheit (82) zum Ermitteln einer Repräsentation (CL) eines Volumenobjekts (COT) auf Basis von dreidimensionalen Bilddaten (BD_{3D}), wobei das Volumenobjekt (COT) eine röhrenartige Struktur aufweist und wobei die Repräsentation (CL) des Volumenobjekts (COT) eine Mittellinie längs des Verlaufs der röhrenartigen Struktur umfasst,
- eine Reformatierungseinheit (83) zum Erzeugen einer zweidimensionalen Darstellung (BD_{2D}) unter Anwendung einer nicht-linear planaren Reformatierung (CPR) des Volumenobjekts (COT) auf Basis der ermittelten Repräsentation (CL), wobei die Mittellinie des Volumenobjekts dazu genutzt wird, eine Fläche im dreidimensionalen Raum für die Reformatierung festzulegen, welche die Röhrenwände der röhrenartigen Struktur in ihrem Verlauf längs der Mittellinie ebenfalls umfasst,
- eine Bildevidenz-Editierungseinheit (84) zum Editieren von Grenzflächenindikatoren (SC_{2D}), welche den Oberflächenverlauf des Volumenobjekts (COT) definieren, in der zweidimensionalen Darstellung (BD_{2D}), wobei Grenzflächenindikatoren einem Benutzer als Markierungslinien in der zweidimensionalen Darstellung angezeigt werden, wobei ein Benutzer zum Editieren von Grenzflächenindikatoren (SC_{2D}) Änderungen als Markierungslinien in der zweidimensionalen Darstellung einzeichnet oder in der zweidimensionalen Darstellung dargestellte Markierungslinien verschiebt und in ihrer Gestalt verändert,
- eine Rücktransformationseinheit (85) zum Erzeugen einer dreidimensionalen Darstellung (SC_{3D}) der in der zweidimensionalen Darstellung editierten Grenzflächenindikatoren (SC_{2D}) durch Rücktransformieren der editierten Grenzflächenindikatoren (SC_{2D}) in den dreidimensionalen Raum,
- eine Modellierungseinheit (86) zum Erzeugen einer Modelldarstellung des Volumenobjekts (COT) auf Basis der durch Rücktransformieren erzeugten dreidimensionalen Darstellung der editierten Grenzflächenindikatoren (SC_{2D}) im dreidimensionalen Raum.

10. Medizinische bildgebende Einrichtung, vorzugsweise CT-System, aufweisend:
- eine Scaneinheit zum Abtasten eines Untersuchungsbereichs eines zu untersuchenden Objekts,
- eine Steuerungseinrichtung zum Ansteuern der Scaneinheit,
- eine Volumenobjekt-Modellierungseinrichtung (80) nach Anspruch 9.

11. Computerprogrammprodukt mit einem Computerprogramm, welches direkt in eine Speichereinrichtung einer Steuerungseinrichtung einer medizinischen bildgebenden Einrichtung ladbar ist, mit Programmabschnitten, um alle Schritte des Verfahrens nach einem der Ansprüche 1 bis 8 auszuführen, wenn das Computerprogramm in der Steuerungseinrichtung der medizinischen bildgebenden Einrichtung ausgeführt wird.

12. Computerlesbares Medium, auf welchem von einer Rechnereinheit einlesbare und ausführbare Programmabschnitte gespeichert sind, um alle Schritte des Verfahrens nach einem der Ansprüche 1 bis 8 auszuführen, wenn die Programmabschnitte von der Rechnereinheit ausgeführt werden.

## Claims

1. Method for interactively generating a geometric model of a volume object (COT) on the basis of three-dimensional image data (BD_{3D}) of an examination region of interest of an examination subject, wherein the volume object (COT) has a tubular structure, comprising the steps of:
- determining a representation (CL) of the volume object (COT) on the basis of three-dimensional image data (BD_{3D}) of the volume object (COT), wherein the representation (CL) of the volume object (COT) comprises a centreline along the course of the tubular structure,
- generating a two-dimensional representation (BD_{2D}) using a non-linear planar reformation (CPR) of the volume object (COT) on the basis of the determined representation (CL), wherein the centreline of the volume object is used to specify a surface in three-dimensional space for the reformation, which surface also comprises the tube walls of the tubular structure in their course along the centreline,
- editing boundary indicators (SC_{2D}), which define the surface profile of the volume object (COT), in the two-dimensional representation (BD_{2D}), wherein boundary indicators (SC_{2D}) are displayed to a user as marking lines in the two-dimensional representation, wherein, to edit boundary indicators (SC_{2D}), a user draws in changes as marking lines in the two-dimensional representation or moves marking lines displayed in the two-dimensional representation and changes their shape,
- generating a three-dimensional representation (SC_{3D}) of the edited boundary indicators (SC_{2D}) by back-transforming the boundary indicators (SC_{2D}) edited in the two-dimensional representation into three-dimensional space, and
- determining a geometric model-based representation of the volume object (COT) on the basis of the three-dimensional representation, generated by back-transformation, of the edited boundary indicators (SC_{2D}) in three-dimensional space.

2. Method according to claim 1, wherein the volume object (COT) has a hollow organ.

3. Method according to claim 2, wherein the tubular structure comprises a three-dimensional vascular structure, preferably a coronary vascular structure.

4. Method according to claim 2 or 3, wherein the centreline (CL) defines the course of the tubular structure.

5. Method according to one of claims 2 to 4, wherein the edited boundary indicators (SC_{2D}, SC_{3D}) mark the course of tube walls of the tubular structure.

6. Method according to one of claims 1 to 5, wherein the back-transformation of the edited boundary indicators (SC_{2D}) from the two-dimensional representation (BD_{2D}) into three-dimensional space is based on an inversion of the non-linear planar reformation (CPR).

7. Method according to one of claims 1 to 6, wherein the modelled volume object (COT) is represented pictorially by means of a surface network in three-dimensional space and/or by means of intersection lines lying in the reformatted surface of the two-dimensional representation (BD_{2D}).

8. Method according to claim 7, wherein for the case of a model-based representation of the volume object (COT) by means of two-dimensional intersection lines lying in the reformatted surface, said intersection lines are used as candidate boundary indicators for the purpose of refining the model in a repeated step of editing boundary indicators (SC_{2D}) performed within the scope of an iteration.

9. Volume object modelling device (80), comprising:
- a representation determination unit (82) for determining a representation (CL) of a volume object (COT) on the basis of three-dimensional image data (BD_{3D}), wherein the volume object (COT) has a tubular structure and wherein the representation (CL) of the volume object (COT) comprises a centreline along the course of the tubular structure,
- a reformation unit (83) for generating a two-dimensional representation (BD_{2D}) using a non-linear planar reformation (CPR) of the volume object (COT) on the basis of the determined representation (CL), wherein the centreline of the volume object is used to specify a surface in three-dimensional space for the reformation, which surface also comprises the tube walls of the tubular structure in their course along the centreline,
- an image evidence editing unit (84) for editing boundary indicators (SC_{2D}) which define the surface profile of the volume object (COT) in the two-dimensional representation (BD_{2D}), wherein boundary indicators are displayed to a user as marking lines in the two-dimensional representation, wherein, to edit boundary indicators (SC_{2D}), a user draws in changes as marking lines in the two-dimensional representation or moves marking lines displayed in the two-dimensional representation and changes their shape,
- a back-transformation unit (85) for generating a three-dimensional representation (SC_{3D}) of the boundary indicators (SC_{2D}) edited in the two-dimensional representation by back-transforming the edited boundary indicators (SC_{2D}) into three-dimensional space,
- a modelling unit (86) for generating a model-based representation of the volume object (COT) on the basis of the three-dimensional representation, generated by back-transformation, of the edited boundary indicators (SC_{2D}) in three-dimensional space.

10. Medical imaging device, preferably CT system, comprising:
- a scan unit for scanning an examination region of interest of a subject that is to be examined,
- a control device for controlling the scan unit,
- a volume object modelling device (80) according to claim 9.

11. Computer program product having a computer program which can be loaded directly into a memory device of a control device of a medical imaging device, and having program sections for carrying out all steps of the method according to one of claims 1 to 8 when the computer program is executed in the control device of the medical imaging device.

12. Computer-readable medium on which program sections which can be read and executed by a computer unit are stored in order to carry out all steps of the method according to one of claims 1 to 8 when the program sections are executed by the computer unit.

## Revendications

1. Procédé de production interactive d'un modèle géométrique d'un objet (COT) ayant du volume à l'aide de données (BD_{3D}) d'image en trois dimensions d'une partie en examen d'un objet en examen, dans lequel l'objet (COT) ayant du volume a une structure de type tubulaire, comportant les stades :
- établissement d'une représentation (CL) de l'objet (COT) ayant du volume sur la base de données (BD_{3D}) d'image en trois dimensions de l'objet (COT) ayant du volume, dans lequel la représentation (CL) de l'objet (COT) ayant du volume comprend une ligne médiane le long du tracé de la structure de type tubulaire,
- production d'un représentation (BD_{2D}) en deux dimensions en appliquant un reformatage (CPR) planaire non linéaire de l'objet (COT) ayant du volume sur la base de la représentation (CL) établie, dans lequel on utilise la ligne médiane de l'objet ayant du volume pour fixer une surface dans l'espace en trois dimensions pour le reformatage, qui comprend également les parois de la structure de type tubulaire dans son tracé le long de la ligne médiane,
- édition d'indicateurs (SC_{2D}) de surfaces limites, qui définissent le tracé de surface de l'objet (COT) ayant du volume dans la représentation (BD_{2D}) en deux dimensions, dans lequel des indicateurs de surfaces limites sont indiqués à l'utilisateur sous la forme de lignes de repérage dans la représentation en deux dimensions, dans lequel un utilisateur dessine, pour l'édition d'indicateurs (SC_{2D}) de surfaces limites, des modifications comme lignes de repérage dans la représentation en deux dimensions ou décale des lignes de repérage représentées dans la représentation en deux dimensions et modifie leur forme,
- production d'une représentation (SC_{3D}) en trois dimensions des indicateurs (SC_{2D}) de surfaces limites édités, par retransformation des indicateurs (SC_{2D}) de surfaces limites édités dans la représentation en deux dimensions, dans l'espace en trois dimensions,
- établissement d'une représentation géométrique modèle de l'objet (COT) ayant du volume dans l'espace en trois dimensions, sur la base de la représentation en trois dimensions, produite par la retransformation, des indicateurs (SC_{2D}) de surfaces limites édités.

2. Procédé suivant la revendication 1, dans lequel l'objet (COT) ayant du volume comporte un organe creux.

3. Procédé suivant la revendication 2, dans lequel la structure de type tubulaire comprend une structure de vaisseau en trois dimensions, de préférence une structure de vaisseau coronaire.

4. Procédé suivant la revendication 2 ou 3, dans lequel la ligne (CL) médiane définit le tracé de la structure de type tubulaire.

5. Procédé suivant l'une des revendications 2 à 4, dans lequel les indicateurs (SC_{2D}, SC_{3D}) de surfaces limites édités repèrent le tracé de parois de la structure de type tubulaire.

6. Procédé suivant l'une des revendications 1 à 5, dans lequel la retransformation des indicateurs (SC_{2D}) de surfaces limites édités, de la représentation (BD_{2D}) en deux dimensions à l'espace en trois dimensions, repose sur une inversion du reformatage (CPR) planaire non linéaire.

7. Procédé suivant l'une des revendications 1 à 6, dans lequel on représente en image l'objet (COT) modélisé ayant du volume par un réseau de surfaces dans l'espace en trois dimensions et/ou par des lignes de coupe se trouvant dans la surface reformatée de la représentation (BD_{2D}) en deux dimensions.

8. Procédé suivant la revendication 7, dans lequel, dans le cas d'une représentation par un modèle de l'objet (COT) ayant du volume, avec des lignes de coupe en deux dimensions se trouvant dans la surface reformatée, on utilise ces lignes de coupe pour un raffinement du modèle comme indicateurs de surfaces limites candidats lors d'un stade exécuté à nouveau dans le cadre d'une itération de l'édition d'indicateurs (SC_{2D}) de surfaces limites.

9. Dispositif (80) de modélisation d'un objet ayant du volume, comportant :
- une unité (82) d'établissement d'une représentation pour l'établissement d'une représentation (CL) d'un objet (COT) ayant du volume sur la base de données (BD_{3D}) en trois dimensions, dans lequel l'objet (COT) ayant du volume a une structure de type tubulaire et dans lequel la représentation (CL) de l'objet (COT) ayant du volume comprend une ligne médiane le long du tracé de la structure de type tubulaire,
- une unité (83) de reformatage pour la production d'une représentation (BD_{2D}) en deux dimensions en appliquant un reformatage (CPR) planaire non linéaire de l'objet (COT) ayant du volume sur la base de la représentation (CL) établie, dans lequel on utilise la ligne médiane de l'objet ayant du volume pour fixer pour le reformatage une surface dans l'espace en trois dimensions, qui comprend également les parois de la structure de type tubulaire dans son tracé le long de la ligne médiane,
- une unité (84) d'édition de preuves d'image pour l'édition d'indicateurs (SC_{2D}) de surfaces limites, qui définissent le tracé de surface de l'objet (COT) ayant du volume dans la représentation (BD_{2D}) en deux dimensions, dans lequel des indicateurs de surfaces limites sont indiqués à un utilisateur sous la forme de lignes de repérage dans la représentation en deux dimensions, dans lequel un utilisateur dessine, pour l'édition d'indicateurs (SC_{2D}) de surfaces limites, des modifications comme lignes de repérage dans la représentation en deux dimensions ou décale des lignes de repérage représentées dans la représentation en deux dimensions et les modifie dans leur forme,
- une unité (85) de retransformation pour la production d'une représentation (SC_{3D}) en trois dimensions des indicateurs (SC_{2D}) de surfaces limites édités dans la représentation en deux dimensions en retransformant, dans l'espace en trois dimensions, les indicateurs (SC_{2D}) de surfaces limites édités,
- une unité (86) de modélisation pour la production, dans l'espace en trois dimensions, d'une représentation par modèle de l'objet (COT) ayant du volume sur la base de la représentation en trois dimensions produite par la retransformation des indicateurs (SC_{2D}) de surfaces limites édités.

10. Dispositif d'imagerie médicale, de préférence système de tomodensitométrie assistée par ordinateur, comportant :
- une unité de balayage pour le balayage d'une région en examen d'un objet à examiner,
- un dispositif de commande pour la commande de l'unité de balayage,
- un dispositif (80) de modélisation d'un objet ayant du volume suivant la revendication 9.

11. Produit de programme d'ordinateur comprenant un programme d'ordinateur, qui peut être chargé directement dans un dispositif de mémoire d'un dispositif de commande d'un dispositif d'imagerie médicale, comprenant des parties de programme, pour exécuter tous les stades du procédé suivant l'une des revendications 1 à 8, lorsque le programme d'ordinateur est exécuté dans le dispositif de commande du dispositif d'imagerie médicale.

12. Support déchiffrable par ordinateur, sur lequel sont mises en mémoire des parties de programme pouvant être déchiffrées et exécutées par une unité informatique, afin d'exécuter tous les stades du procédé suivant l'une des revendications 1 à 8, lorsque les parties du programme sont exécutées par l'unité informatique.
